# EUROPEAN PATENT APPLICATION

(11) **EP 2 327 988 A1**
(43) Date of publication of application: **01.06.2011**
(21) Application number: 09177420.8
(22) Date of filing: 28.11.2009
(51) Int. Cl.: G01N 33/68

(54) **Method for diagnosis of fibrotic diseases**

(71) Applicant: Assistance Publique Hôpitaux De Paris, 75004 Paris 4 (FR)
(72) Inventor: The designation of the inventor has not yet been filed
(74) Representative: Flesselles, Bruno F.G.

(57) **Abstract**

The present invention relates to osteoprotegerin as a new serum marker useful by itself or in combination with other markers for diagnosis liver fibrosis or cirrhosis in a patient.

## Description

The present invention relates to osteoprotegerin as a new serum marker useful by itself or in combination with other markers for diagnosis liver fibrosis or cirrhosis in a patient. The invention is also drawn to diagnosis kits for the implementation of the diagnosis method.

In the last few years, different diagnosis methods have been developed to allow detection of liver disease through measurement of serum markers, in place of lover biopsy.

This made it possible to reduce the number of biopsies, previously considered as mandatory for the management of patients with liver disease, (in particular in patients infected by the hepatitis C virus (HCV)), particularly for the staging of fibrosis.

Liver biopsy is indeed an aggressive procedure. Although numerous studies had shown significant predictive values of several individual markers for the diagnosis of cirrhosis, the combination of markers as developed recently gives better predictive results, including for the diagnosis of earlier stage as few septa (beginning of bridging fibrosis).

The *in vitro* methods developed recently make it possible to to detect early stages in the development of liver pathology, in order to improve the patient treatment, and the follow-up of the disease. These tests have a quite good predictive value of the level of fibrosis in the patient, but could be improved in order to have better sensibility and/or specificity.

Sensibility is the probability of obtaining a positive result if the disease is present. A test that is 100 % sensible has thus no false negative.

Specificity is the probability of obtaining a negative result if the disease is absent. A test that is 100 % specific has thus no false positive.

It is always important to have a test that is both specific and sensitive.

The quality of a diagnosis test may be determined by drawing a Receiving Operating Characteristic (ROC) curve and measuring the Area Under Receiving Operating Characteristic curve (AUROC).

The ROC curve is drawn by plotting the sensitivity versus (1-specificity), after classification of the patients, according to the result obtained for the diagnosis test, for different thresholds (from 0 to 1).

It is usually acknowledged that a ROC curve the area under which has a value superior to 0.7 is a good predictive curve for diagnosis. The ROC curve has to be acknowledged as a curve allowing prediction of the quality of a diagnosis test.

As indicated above, different *in vitro* diagnosis methods have been described in the art, to measure the staging of liver fibrosis and of cirrhosis.

One can cite WO 2002/016949, WO 2006/082522, WO 2006/103570, WO 2006/015874, WO 2006/015873, or WO 2005/116901.

Different scientific publications also describe use of biochemical markers for assessing liver disease: Halfon et al. (J Hepatol. 2007 Mar;46(3):395-402), Calès et al. (Hepatology. 2005 Dec;42(6):1373-81), Adams et al. (Clin Chem. 2005 Oct;51(10):1867-73), Imbert-Bismut et al. (Lancet. 2001 Apr 7;357(9262):1069-75).

Some *in vitro* tests have been tested and assessed by the Haute Autorité de Santé (French Authority of Health).

The Fibrotest® combines five markers: alpha2-macroglobulin, haptoglobin, apolipoprotein A1, total bilirubin and gamma-glutamyl transpeptidase, and adjusts with sex and age. The algorithm for Fibrotest® is confidential, but results can be obtained from the company Biopredictive.

The FibroMetre® combines six markers: platelets number, prothrombin level, aspartate amino-transferase, alpha2-macroglobulin, hyaluronic acid and urea and adjusts with sex and age. The algorithm for FibroMètre® is confidential, but results can be obtained from the company BioLiveScale.

Hepascore combines four markers: alpha2-macroglobulin, hyaluronic acid, total bilirubin and gamma-glutamyl transpeptidase and ajusts with sex and age. The Hepascore formula is y/1+y, avec y = exp [- 4,185818 - 0,0249 x age (years) + 0,7464 x sex (1 for male and 0 for female) + 1,0039 x alpha2-macroglobulin (g/1) + 0,0302 x hyaluronic acid (µg/l) + 0,0691 x total bilirubin (µg/l) - 0,0012 x gamma-glutamyl transpeptidase (Ul/l)].

The ELF score combines three markers: hyaluronic acid, N-terminal of type III pro-collagen, and Tissue inhibitor metalloproteinase type-1 (TIMP-1) and adjusts for age. The ELF formula is: - 0,014 In(age) + 0,616 In(hyaluronic acid) + 0,586 In(N-terminal of type III pro-collagen) + 0,472 In(TIMP-1 ) - 6,38.

After liver infection (such as infection by a hepatitis virus, and in particular by the hepatitis C virus) or liver damage (such as damage due to alcoholism), the evolution of the disease can lead to fibrosis, and later to cirrhosis. The liver biopsy allows for the determination of the stage of the fibrosis, but also the presence of liver necroinflammatory lesions.

The intensity and activity of such lesions, in complement to the degree of fibrosis, are acknowledged by physicians as an important factor for diagnosis and prognosis of the evolution of the disease, and in order to determine the type of treatment to administrate, in particular for HCV infection.

The inventors have demonstrated that osteoprotegerin (OPG, tumor necrosis factor receptor superfamily member 11 b, NM_002546) is a good marker of fibrosis and cirrhosis, either by itself, or in combination with other serum markers. Measurement of the level of OPG from a sample of blood that has been harvested from patient should therefore be contemplated in *in vitro* diagnosis methods aiming to determine extend of a liver disease.

In particular, introduction of OPG as a marker to be measured makes it possible to obtain a better AUROC, thus indicating improvement of the diagnostic test.

Circulating level of osteoprotegerin has been described as being elevated in some fibrotic diseases, as indicated by Guarlabens et al. (J Bone Miner Metab. 2009;27(3):347-54), Garcia-Valdecasas-Campelo et al. (Alcohol Alcohol. 2006 May-Jun;41(3):261-6), Moschen et al., J Hepatol. 2005 Dec;43(6):973-83), Fábrega et al. (Liver Int. 2005 Apr;25(2):305-10) or Szalay et al. (J Hepatol. 2003 Apr;38(4):395-400).

In particular, serum OPG level is elevated in chronic alcohol liver disease. It is postulated that this may be due to enhanced bone loss, as ethanol decreases bone formation in a dose-dependent fashion (see Garcia-Valdecasas-Campelo et al., *op.cit.).*

Nevertheless, the fact that OPG serum concentration may be used as a marker of the level of liver fibrosis, in particular for patients having HCV disease, has never be demonstrated, nor suggested.

The invention now provides a method of diagnosis (preferably performed *in vitro)* well suited for diagnosis of significant fibrosis (from few septa to cirrhosis) and/or liver necroinflammatory lesions.

### DESCRIPTION OF THE FIGURES

Figure 1: correlation between the OPG serum concentration and the fibrotic stage. Horizontal line: median; error bars: extreme measured values; rectangle: 25^{tn} percentile
Figure 2 : comparison of the AUROC (Area under ROC curve) for the different non-invasive diagnosis available on the French market. A. AUROC for significant fibrosis (F2, F3 or F4). B. AUROC for cirrhosis. Score 1 corresponds to function f1; Score 2 corresponds to function f2.

The present invention is drawn to a method for diagnosis of liver fibrosis and/or presence of liver necroinflammatory lesions in a patient comprising the steps of : a) measuring the values of osteoprotegerin in the serum or plasma of said patient, wherein a concentration of osteoprotegerin in the serum or plasma that is higher than 1.5 µg/l is indicative of a stage of fibrosis that is equal or higher than the F2 according to the METAVIR classification.

The invention also relates to the use of osteoprotegerin as a biochemical marker in a non-invasive method of diagnostic of a liver disease (fibrosis and/or cirrhosis).

In a specific embodiment, the invention is drawn a method for diagnosis of liver fibrosis and/or presence of liver necroinflammatory lesions in a patient comprising the steps of :
a) measuring the values of at least three biochemical markers in the serum or plasma of said patient,
b) combining said values through a logistic function including said markers and,
c) determining the presence of liver fibrosis and/or liver necroinflammatory lesions in said patient, depending on the end value of said logistic function.
wherein one of said biochemical markers is osteoprotegerin.

The biochemical markers can also been assessed in the plasma of the patients. The method is an *in vitro* method, performed on a biological sample that has beforehand been harvested from a patient.

The logistic function may be obtained through the following method:
i) classification of the patients in different groups according to the extend of their disease;
ii) identification of factors which differ significantly between these groups by unidimensional analysis;
iii) logistic regression analysis to assess the independent discriminative value of markers for the diagnosis of fibrosis and/or liver necroinflammatory lesions
iv) construction of the logistic function by combination of these identified independent factors (construction of an index).

By definition the best index ("fibrosis score") in term of discrimination was the logistic regression function combining the independent factors.

The logistic function is obtained by combining the relative weight of each parameter, as individually determined in the logistic regression, with a negative sign when the markers harbor a negative correlation with the stage of fibrosis.

Logarithms (neperian logarithm) are preferably used for markers whose values have a very large range.

As indicated above, the quality of the logistic function (which represents the quality of the diagnosis test) is analyzed with the aid of a ROC curve, that is obtained depending of the threshold desired for the diagnosis. The way of obtaining the ROC curve is well known in the art.

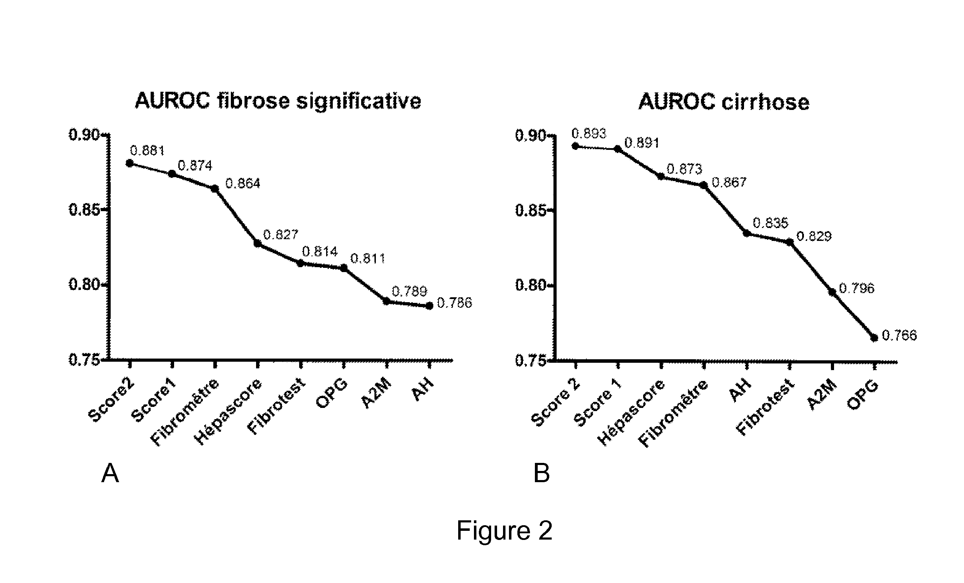

The patients may be classified depending on the presence of fibrosis, starting at few septa, but classification could be changed if diagnosis of patient only with a large number of septa or with cirrhosis is intended. This would lead to another ROC curve, as shown in Figure 2A and 2B.

The diagnosis of the presence of liver fibrosis and/or liver necroinflammatory lesions in the patient can be further refined by the data concerning the expected prevalence of liver fibrosis in the population.

Preferably, the biochemical markers that are dosed in step a) of the method according to the present invention are "simple" biochemical markers, which can easily be dosed with methods already known in the art (chromatography, electrophoresis, ELISA dosing...).

Thus, markers (other than osteoprotegerin) that are perfectly suitable for the method of the invention include alpha2-macroglobulin (A2M), alanine aminotransferase (ALT), aspartate aminotransferase (AST), GGT (gamma glutamyl transpeptidase), gamma-globulin, total bilirubin, albumin, alpha1-globulin, alpha2-globulin, haptoglobin, beta-globulin, Apolipoprotein A1 (apoA1), IL10, TGFbeta1, apoA2, apoB, blood platelet number, prothrombin, hyaluronic acid (HA), urea, N-terminal of type III pro-collagen, and Tissue inhibitor metalloproteinase type-1 (TIMP-1).

The list of these markers is not limitative. Other markers may easily be introduced in the logistic function, developed as indicated above.

In a particular embodiment of the method of the invention, at least 4, more preferably 5 or 6, or even more (such as 7 or 10) biochemical markers are studied and dosed in step a) of the method.

In a specific embodiment, the markers used are OPG, ASAT, ApoA1, A2M GGT.

In another embodiment, the markers used are OPG, ASAT, ApoA1, A2M GGT and HA.

In another embodiment, the markers used are OPG, ApoA1, A2M, GGT, haptoglobin and total bilirubin.

In another embodiment, the markers used are OPG, platelets number, prothrombin level, AST, A2M, HA and urea.

In another embodiment, the markers used are OPG, A2M, HA, total bilirubin and GGT.

In another embodiment, the markers used are OPG, HA, N-terminal of type III pro-collagen, and Tissue inhibitor metalloproteinase type-1 (TIMP-1).

The choice of the markers is within the skills of the person skilled in the art, as long as said markers show a correlation with the fibrotic (or cirrhotic) stage of the patient. Thus, any combination of the above markers may be used in the context of the invention (as long as the function also includes OPG).

A2M or HA are usually used, as they are already known strong markers of liver malfunctioning.

AST or ALT can indifferently be used as these markers are correlated (WO 02/016949). Therefore, replacement of one marker by the other only results in the balancing of the coefficient in the logistic function, taking the correlation factor into account.

The logistic function preferably takes the age and / or the gender of the patient into account. The different coefficients used for the values obtained for the different markers in the logistic function can be calculated through statistical analysis, as described in WO 02/016949.

In particular, suitable logistic functions that can be used for the implementation of the method of the invention are as follows:
f1 = 1/(1+y) avec y = exp[-(a1 + a2 x [Age (years)] + a3 x [Sex (female=0, male=1)]) + a4 x In([alpha2-macroglobulin (g/L)]) + a5 x In([osteoprotegerin (µg/L)]) + a6 x In([ASAT (Ul/L)]) - (a7 x [ApoA1 (g/L)]) + a8 x In([GGT (Ul/L)]))], with
   - a1 comprised between -3 and -4.5,
   - a2 comprised between 0.004 and 0.01,
   - a3 comprised between 0.05 and 0. 08,
   - a4 comprised between 1.20 and 1.28,
   - a5 comprised between 0.4 and 0.6,
   - a6 comprised between 0.68 and 0.78,
   - a7 comprised between 1.4 and 1.7,
   - a8 comprised between 0.32 and 0.36
f2 = 1/(1+y) avec y = exp[-(a1 + a2 x [Age (years)] + a3 x [Sex (female=0, male=1)]) + a4 x In([alpha2-macroglobulin (g/L)]) + a5 x In([osteoprotegerin (µg/L)]) + a6 x In([ASAT (Ul/L)]) - (a7 x [ApoA1 (g/L)]) + a8 x [GGT (Ul/L)] + a9 x In([AH (µg/L)]))], with
   - a1 comprised between -3 and -4.5,
   - a2 comprised between 0.004 and 0.01,
   - a3 comprised between 0.05 and 0. 08,
   - a4 comprised between 1.20 and 1.28,
   - a5 comprised between 0.4 and 0.6,
   - a6 comprised between 0.68 and 0.78,
   - a7 comprised between 1.4 and 1.7,
   - a8 comprised between 0.001 and 0.005
   - a9 comprised between 0.2 and 0.25.

Specific usable functions are in particular:
f1 = 1/(1+y) avec y = exp[-(-4.012 + 0.008 x [Age (years)] + 0.057 x [Sex (female=0, male=1)]) + 1.234 x In([A2M (g/L)]) + 0.566 x In([OPG (µg/L)]) + 0.762 x In([ASAT (Ul/L)]) - (1.673 x [ApoA1 (g/L)]) + 0.341 x In([GGT (Ul/L)]))]
f2 = 1/(1+y) avec y = exp[-(-3.655 + 0.005 x [Age (years)] + 0.072 x [Sex (female=0, male=1)]) + 1.264 x In([A2M (g/L)]) + 0.454 x In([OPG (µg/L)]) + 0.7 x In([ASAT (Ul/L)]) - (1.459 x [ApoA1 (g/L)]) + 0.003 x [GGT (Ul/L)] + 0.228 x In([HA (ug/L)]))].

In fact, the numerical definitions for the coefficients in the different functions can slightly vary (about 10-15 %) depending of the number and characteristics of patients studied. Therefore, the value given for the coefficients of the different markers have to be interpreted as capable to being slightly different, without reducing the scope of the invention.

The logistic function may be used for the diagnosis of liver fibrosis, as well as for diagnosis of cirrhosis.

It is indeed possible to draw conclusions about the presence of liver fibrosis for the patient, depending of the end value obtained by including the values measured for the biological markers through the logistic function. Conclusion about the presence of cirrhosis is obtained if cirrhosis is chosen as the threshold in the drawing of the ROC curve.

The method of the invention is also usable as a predictive mean for the evolution of the disease. In particular, when the patient is infected with the hepatitis C virus, it is often possible to determine the date of the infection (usually through transfusion). Therefore, the use of the method of the invention to determine the degree of evolution of the disease through the date of the diagnosis can also allow for the prognosis of the future development of the disease.

The data obtained by the diagnosis method according to the invention can also be very valuable for the physician to choose a suitable treatment for the patient, according to the stage of the disease.

Repeating the method of the invention on a regular basis also makes it possible to determine the efficiency of a treatment.

Depending of the prevalence of liver fibrosis in the population of patients that are consulting, the data obtained with the method of the invention can be used as an help to determine whether it is necessary to perform a liver biopsy on the patient.

The method of the invention is intended to be used for patient suffering of liver fibrosis, which could develop to cirrhosis. In particular, the method of the invention is advantageously performed for detecting liver fibrosis in patient suffering form a disease included in the group consisting of hepatitis B and C, alcoholism, hemochromatosis, metabolic disease, diabetes, obesity, autoimmune hepatitis, primary biliary cirrhosis, alpha1-antitrypsin deficit, Wilson disease.

The method of the invention is best performed on patients infected with a hepatitis virus, in particular the hepatitis C virus.

The invention is also drawn to a kit of diagnosis of liver fibrosis and/or liver necroinflammatory lesions in a patient, comprising instructions allowing to determine the presence of liver fibrosis and/or liver necroinflammatory lesions in said patient, after dosage of biochemical markers.

The instructions may comprise the logistic function that has to be use after determination of the dosage of the biochemical markers. It can appear as a printed support as well as a computer usable support, such as a software. The instructions may also comprise the ROC curve depending of the threshold that is looked for, to allow the analysis of the end data obtained from the logistic function. They may also comprise different tables that permit to obtain the predictive values, depending of the expected prevalence of fibrosis in the patient population.

The diagnosis kit according to the present invention may also contain elements allowing the dosage of the biological markers of interest.

The method of the invention can easily be automated, the dosage of the markers being performed automatically, the data being sent to a computer or a calculator that will calculate the value of the logistic function and analyze it with the aid of the ROC curve, and eventually the prevalence of liver fibrosis in the patient population. The data obtained by the physician is therefore more easily interpretable, and will allow for an improvement in the process for deciding the need of a biopsy or the adequate treatment to prescribe.

The following examples are meant to describe an aspect of invention, but shall not be limiting the invention. In particular, the logistic functions mentioned in the examples are only an illustration of the quality of results that are obtained when using OPG as a biological marker in such diagnosis methods.

### EXAMPLES

### Example 1: Use of OPG alone as a marker of liver fibrosis

OPG level was measured in serum of 139 patients with chronic HCV infection, from the Fibrostar group (patients having no treatment), as well as in 21 control patients without liver pathologies.

The patients were classified as follows after liver biopsy (METAVIR classification): FO = 12,2 %, F1= 30,2 %, F2= 18,7 %, F3= 19,4 %, F4= 19,4 %.

Serum concentration of OPG is significantly correlated with the fibrosis stage as evaluated by liver biopsy (correlation coefficient of 0,55 (0,42-0,66) (corrélation de Spearman, p<0,0001)).

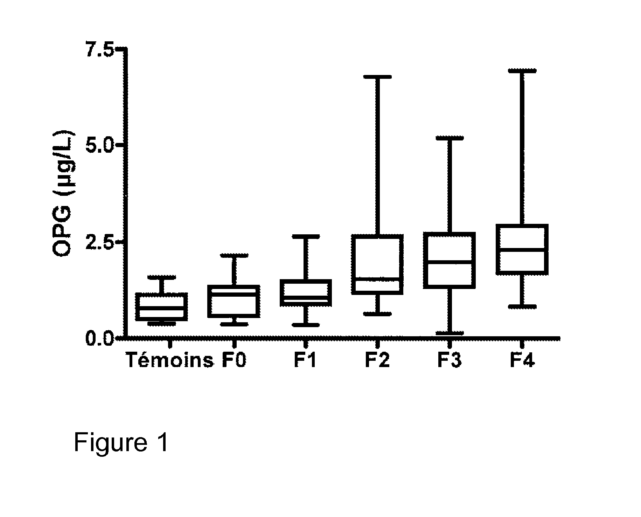

Figure 1 shows the progression of the concentration of OPG depending on the fibrosis stage. It is thus possible to see that a concentration of OPG higher than 1.5 µg/l indicates significant fibrosis.

### Example 2: Use of OPG in combination with other markers

Methods: For experimental study, OPG mRNA was measured using quantitative RT-PCR in total liver of 24 rats submitted to bile duct ligation and in a culture of rat HSCs at different activation stages.

For human clinical study, serum osteoprotegerin was determined in serum of 139 untreated chronic HCV patients from the Fibrostar cohort using an ELISA kit ("human OPG/TNRFSF11 B", R&D systems) simultaneously with others serum fibrosis markers. All patients had liver biopsy (at least 15 mm and/or at least 11 portal tracts).

AUROC of Fibrotest, Fibrometer, Hepascore, hyaluronate (HA), a2-macroglobulin (a2M), OPG and two new fibrosis indices including serum OPG, age, sex, a2M, ASAT, apolipoprotein Al, GGT ± HA, were compared for the diagnosis of significant fibrosis (METAVIR F2 or more) and cirrhosis (METAVIR = F4).

Results: OPG mRNA was elevated in fibrotic total liver from BDL rats and only slightly decreased in HSCs as they transdifferentiate to the myofibroblastic phenotype.

In HCV patients, OPGscores have more elevated AUROC than others scores and markers for the diagnosis of significant fibrosis and cirrhosis (Figure 2). Conclusion: OPG is overexpressed when liver fibrosis occurs. Increasing serum OPG could be related to increasing mRNA synthesis in the fibrotic liver by MF-HSCs but also by others liver cell types. Fibrosis scores including OPG could be more efficient to diagnose significant fibrosis and cirrhosis than Fibrometer, Fibrotest or Hepascore.

The new fibrotic indices were generated as follows:
According to the METAVIR scoring system, patients were divided into several groups.
F0 = no fibrosis
F1 = portal fibrosis without septa
F2 = portal fibrosis with some septas
F3 = septal fibrosis without cirrhosis
F4 = cirrhosis

The major endpoint was the identification of patients with significant fibrosis (F2, F3 or F4) versus patients without significant fibrosis (FO or F1).

The first stage consisted of identifying factors which differed significantly between these groups by unidimensional analysis using the chi-square, Student t test or Mann-Whitney test.

The second stage consisted of logistic regression analysis to assess the independent discriminative value of markers for the diagnosis of fibrosis.

The third step was to construct an index combining these identified independent factors. By definition the best index ("fibrosis score") in term of discrimination was the logistic regression function combining the independent factors.

The diagnostic values of these indexes and of the isolated factors were assessed by sensitivity, specificity, positive and negative predictive values and receiver operating characteristics.

The predictive values were assessed for the observed prevalence of significant fibrosis, but also for lower or higher prevalence.

The respective overall diagnostic values were compared by the area under the Receiver Operating Characteristic curves. The ROC curve is drawn by plotting the sensitivity versus (1-specificity), after classification of the patients, according to the value obtained for the logistic function, for different thresholds (from 0 to 1).

Score 1 and Score 2 were thus obtained, respectively corresponding to f1 and f2: f1 = 1/(1+y) avec y = exp[-(-4.012 + 0.008 x [Age (years)] + 0.057 x [Sex (female=0, male=1)]) + 1.234 x In([A2M (g/L)]) + 0.566 x In([OPG (µg/L)]) + 0.762 x In([ASAT (Ul/L)]) - (1.673 x [ApoA1 (g/L)]) + 0.341 x In([GGT (Ul/L)]))]
f2 = 1/(1+y) avec y = exp[-(-3.655 + 0.005 x [Age (years)] + 0.072 x [Sex (female=0, male=1)]) + 1.264 x In([A2M (g/L)]) + 0.454 x In([OPG (µg/L)]) + 0.7 x In([ASAT (Ul/L)]) - (1.459 x [ApoA1 (g/L)]) + 0.003 x [GGT (Ul/L)] + 0.228 x In([HA (µg/L)]))].

It is to be noted that the data presented in Figure 2 demonstrates that OPG alone seems as efficient as Fibrotest® for diagnosis of significant fibrosis, and is better than A2M or HA.

The two developed new functions which include OPG and "conventional" markers are more efficient than the available tests for both significant fibrosis and cirrhosis diagnosis.

## Claims

1. A *in vitro* method for diagnosis of liver fibrosis and/or presence of liver necroinflammatory lesions in a patient comprising the steps of : a) measuring the values of at least three biochemical markers in the serum or plasma of said patient, b) combining said values through a logistic function including said markers and, c) analyzing the end value of said logistic function in order to determine the presence of liver fibrosis and/or liver necroinflammatory lesions in said patient, wherein one of said biochemical markers is osteoprotegerin (OPG).

2. The method of claim 1, wherein at least 4 biochemical markers are studied in step a).

3. The method of claim 1, wherein said biochemical markers other than osteoprotegerin are chosen in the group consisting of alpha2-macroglobulin (A2M), alanine aminotransferase (ALT), aspartate aminotransferase (AST), GGT (gamma glutamyl transpeptidase), gamma-globulin, total bilirubin, albumin, alpha1-globulin, alpha2-globulin, haptoglobin, beta-globulin, apoA1, IL10, TGFbeta1, apoA2, apoB, blood platelet number, prothrombin, hyaluronic acid (HA), urea, N-terminal of type III pro-collagen, and Tissue inhibitor metalloproteinase type-1 (TIMP-1).

4. The method of claim 1, wherein the logistic function further takes the age and/or the gender of the patient into account.

5. The method of any of claims 1 to 4, wherein said measured biochemical markers used include a2-macroglobulin, GGT, ASAT, apoAl and osteoprotegerin.

6. The method of any of claims 1 to 5, wherein the logistic function reads:
- f1 = 1/(1+y) avec y = exp[-(a1 + a2 x [Age (years)] + a3 x [Sex (female=0, male=1)]) + a4 x In([alpha2-macroglobulin (g/L)]) + a5 x In([osteoprotegerin (µg/L)]) + a6 x In([ASAT (Ul/L)]) - (a7 x [ApoA1 (g/L)]) + a8 x In([GGT (Ul/L)]))], with
- a1 comprised between -3 and -4.5,
- a2 comprised between 0.004 and 0.01,
- a3 comprised between 0.05 and 0. 08,
- a4 comprised between 1.20 and 1.28,
- a5 comprised between 0.4 and 0.6,
- a6 comprised between 0.68 and 0.78,
- a7 comprised between 1.4 and 1.7,
- a8 comprised between 0.32 and 0.36
- f2 = 1/(1+y) avec y = exp[-(a1 + a2 x [Age (years)] + a3 x [Sex (female=0, male=1)]) + a4 x In([alpha2-macroglobulin (g/L)]) + a5 x In([osteoprotegerin (µg/L)]) + a6 x In([ASAT (Ul/L)]) - (a7 x [ApoA1 (g/L)]) + a8 x [GGT (Ul/L)] + a9 x In([AH (µg/L)]))], with
- a1 comprised between -3 and -4.5,
- a2 comprised between 0.004 and 0.01,
- a3 comprised between 0.05 and 0. 08,
- a4 comprised between 1.20 and 1.28,
- a5 comprised between 0.4 and 0.6,
- a6 comprised between 0.68 and 0.78,
- a7 comprised between 1.4 and 1.7,
- a8 comprised between 0.001 and 0.005
- a9 comprised between 0.2 and 0.25.

7. The method of claim 6, wherein the logistic function is chosen in the group consisting of :
- f1 = 1/(1+y) avec y = exp[-(-4.012 + 0.008 x [Age (years)] + 0.057 x [Sex (female=0, male=1)]) + 1.234 x In([alpha2-macroglobulin (g/L)]) + 0.566 x In([osteoprotegerin (µg/L)]) + 0.762 x In([ASAT (Ul/L)]) - (1.673 x [ApoA1 (g/L)]) + 0.341 x In([GGT (Ul/L)]))]
- f2 = 1/(1+y) avec y = exp[-(-3.655 + 0.005 x [Age (years)] + 0.072 x [Sex (female=0, male=1)]) + 1.264 x In([alpha2-macroglobulin (g/L)]) + 0.454 x In([osteoprotegerin (µg/L)]) + 0.7 x In([ASAT (Ul/L)]) - (1.459 x [ApoA1 (g/L)]) + 0.003 x [GGT (Ul/L)] + 0.228 x In([HA (µg/L)]))].

8. The method of any of claims 1 to 7, wherein the end value of the logistic function is used for the diagnosis of cirrhosis.

9. The method of claim any of claims 1 to 7, wherein the end value of the logistic function is used to predict the evolution of the disease.

10. The method of any of claims 1 to 7, wherein the end value of the logistic function is used for the choice of a suitable treatment for the patient.

11. The method of claim any of claims 1 to 7, wherein the end value of the logistic function is used in the decision of performing a liver biopsy on said patient.

12. The method of any of claims 1 to 7, wherein said patient suffers from a disease involving liver fibrosis, optionally developing to cirrhosis.

13. The method of claim 12, wherein said disease is included in the group consisting of hepatitis B and C, alcoholism, hemochromatosis, metabolic disease, diabetes, obesity, autoimmune liver disease, primary biliary cirrhosis, alpha1-antitrypsin deficit, Wilson disease.

14. A kit of diagnosis of liver fibrosis and/or liver necroinflammatory lesions in a patient, comprising instructions allowing to determine the presence of liver fibrosis and/or liver necroinflammatory lesions in said patient, after the dosage of biochemical markers.

15. A *in vitro* method for diagnosis of liver fibrosis and/or presence of liver necroinflammatory lesions in a patient comprising the steps of : a) measuring the values of osteoprotegerin in the serum or plasma of said patient, wherein a concentration of osteoprotegerin in the serum or plasma that is higher than 1.5 µg/l is indicative of a stage of significant fibrosis (equal or higher than F2 according to the METAVIR classification).

16. Use of osteoprotegerin as a biochemical marker in a non-invasive method of diagnostic of a liver disease.
